(19) Europäisches Patentamt
European Patent Office
Office européen des brevets

(11) Veröffentlichungsnummer: **0 126 299 B1**

(12) **EUROPÄISCHE PATENTSCHRIFT**

(45) Veröffentlichungstag der Patentschrift: **01.04.87**

(51) Int. Cl.⁴: **C 07 C 118/00**, C 07 C 119/042 // C07C125/073

(21) Anmeldenummer: **84104352.4**

(22) Anmeldetag: **17.04.84**

**ERRATUM**

| DIE TEXTSTELLE :<br>TEXT PUBLISHED :<br>LE PASSAGE SUIVANT : | (SEITE, SPALTE, ZEILE)<br>(PAGE, COLUMN, LINE)<br>(PAGE,COLONNE,LIGNE) | | | LAUTET BERICHTIGT:<br>SHOULD READ :<br>DEVRAIT ETRE LU : |
|---|---|---|---|---|
| oder in einem Kunststoff enthal-tenden Wirbelbett | 2 | 2 | 13/14 | oder in einem Kohlenstoff ent-haltenden Wirbelbett |

Tag der Entscheidung über die Berichtigung / Date of decision on rectification: / Date de décision portant sur modification: 08.08.88

Ausgabe- und Veröffentlichungstag: / Issue and publication date: / Date d'edition et de publication: 28.09.88

Patbl.Nr / EPB no: / Bull. no: 88/39

(19) Europäisches Patentamt
European Patent Office
Office européen des brevets

(11) Veröffentlichungsnummer: **0 126 299**
**B1**

# (12) EUROPÄISCHE PATENTSCHRIFT

(45) Veröffentlichungstag der Patentschrift: **01.04.87**

(51) Int. Cl.⁴: **C 07 C 118/00,** C 07 C 119/042 // C07C125/073

(21) Anmeldenummer: **84104352.4**

(22) Anmeldetag: **17.04.84**

(54) **Mehrstufenverfahren zur Herstellung von Hexamethylen-diisocyanat-1,6 und/oder isomeren aliphatischen Diisocyanaten mit 6 Kohlenstoffatomen im Alkylenrest.**

(30) Priorität: **23.04.83 DE 3314788**

(43) Veröffentlichungstag der Anmeldung: **28.11.84 Patentblatt 84/48**

(45) Bekanntmachung des Hinweises auf die Patenterteilung: **01.04.87 Patentblatt 87/14**

(84) Benannte Vertragsstaaten: **BE DE FR GB IT NL**

(56) Entgegenhaltungen:
**EP - A - 0 018 588**
**EP - A - 0 061 013**

(73) Patentinhaber: **BASF Aktiengesellschaft, *Carl-Bosch-Strasse 38, D-6700 Ludwigshafen (DE)***

(72) Erfinder: **Merger, Franz, Dr., Max-Slevogt-Strasse 25, D-6710 Frankenthal (DE)**
Erfinder: **Towae, Friedrich, Dr., Schwedlerstrasse 118, D-6700 Ludwigshafen (DE)**
Erfinder: **Hellbach, Hans, Stettiner Strasse 14, D-6840 Lampertheim (DE)**
Erfinder: **Isbarn, Gunther, Dr., Studernheimer Strasse 1, D-6700 Ludwigshafen (DE)**
Erfinder: **Koehler, Waldemar, Dr., Max-Slevogt-Strasse 28, D-6710 Frankenthal (DE)**

**EP 0 126 299 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

ACTORUM AG

**Beschreibung**

Die technische Herstellung von Hexamethylendiisocyanat-1,6 beruht auf der Phosgenierung von Hexamethylendiamin zu Hexamethylen-dicarbamidsäurechlorid und dessen thermische Spaltung in Hexamethylen-diisocyanat-1,6 und Chlorwasserstoff. Abgesehen von den schwerwiegenden Umweltschutz- und Sicherheitsproblemen, die der Einsatz von Phosgen mit sich bringt, ist dieses Verfahren noch mit weiteren entscheidenden Nachteilen behaftet. So gelingt die Hexamethylendiisocyanat-1,6-Produktion nur mit recht mässigen Raum-Zeit-Ausbeuten. Neben Hexamethylendiisocyanat-1,6 erhält man ausserdem mehrere Nebenprodukte, von denen das wichtigste – 6-Chlorhexylisocyanat – zudem den Nachteil besitzt, dass es nur mit erheblichem destillativem Aufwand vom Hexamethylen-diisocyanat-1,6 abgetrennt werden kann.

Problematisch bei dieser Verfahrensweise sind der hohe Umsatz von Chlor über Phosgen und Carbamidsäurechlorid in Chlorwasserstoff, die Toxizität des Phosgens sowie die Korrosivität des Reaktionsgemisches und die Labilität der in der Regel eingesetzten Lösungsmittel.

Es hat daher nicht an Versuchen gefehlt, Isocyanate, vorzugsweise aromatische Di- und/oder Polyisocyanate, phosgenfrei herzustellen.

Zur Herstellung von aliphatischen und/oder cycloaliphatischen Di- und/oder Polyurethanen werden gemäss EP-A-0018588 primäre aliphatische und/oder cycloaliphatische Di- und/oder Polyamine mit O-Alkylcarbamidsäureestern in Gegenwart von Alkoholen im Verhältnis von $NH_2$-Gruppen der Amine zu Carbamidsäureestern zu Alkohol von 1:0,8 bis 10:0,25 bis 50 bei Temperaturen von 160 bis 300 °C in Gegenwart oder Abwesenheit von Katalysatoren umgesetzt und das entstehende Ammoniak gegebenenfalls abgetrennt. Die erhaltenen Di- und/oder Polyurethane können gegebenenfalls in die entsprechenden Di- und/oder Polyisocyanate übergeführt werden. Detaillierte Reaktionsbedingungen zur Thermolyse werden in der Publikation nicht offenbart.

Nach Angaben der EP-A-28338 werden aromatische Di- und/oder Polyisocyanate hergestellt nach einem Zweistufenverfahren, wobei in der ersten Reaktionsstufe primäre aromatische Di- und/oder Polyamine mit O-Alkyl-carbamidsäureester in Abwesenheit oder Gegenwart von Katalysatoren und gegebenenfalls Harnstoff und Alkohol zu Aryl-di- und/oder -polyurethanen umgesetzt werden und das dabei gebildete Ammoniak gegebenenfalls abgetrennt wird und die erhaltenen Aryl-di- und/ oder -polyurethane durch thermische Spaltung in der zweiten Reaktionsstufe in aromatische Di- und/oder Polyisocyanate übergeführt werden.

Auf diese Weise können aromatische Di- und/ oder Polyisocyanate ohne Mitverwendung von Phosgen in hohen Ausbeuten hergestellt werden.

Die DE-OS 3108990 beschreibt die Herstellung von Hexamethylen-diisocyanat-1,6 durch thermische Spaltung unter Druck von Hexamethylendiethylurethan-1,6 in Gegenwart von Dibenzyltoluol als Lösungsmittel und eines Katalysatorgemisches aus Toluolsulfonsäuremethylester und Diphenylzinndichlorid. Angaben über die Gewinnung der Ausgangskomponente, deren Isolierung sowie die Reinigung und gegebenenfalls Rückgewinnung des Lösungsmittels und der Katalysatormischung werden nicht gemacht. Berechnungen über die Wirtschaftlichkeit des Verfahrens sind daher nicht möglich.

Nach Angaben der Deutschen Patentanmeldung P 3227748.2, P 3248018.0 und P 3142627.1 können Hexamethylen-dialkylurethane katalysatorfrei oder in einem Kunststoff enthaltenden Wirbelbett problemlos in Hexamethylen-diisocyanat-1,6 und Alkohol gespalten werden. Mit diesen Verfahren können jedoch keine Hexamethylen-diisocyanat-Ausbeuten von grösser als 90% erzielt werden, da die Spaltprodukte teilweise rekombinieren. Bei der dadurch erforderlichen Reindestillation des Hexamethylen-diisocyanat-1,6 können sich die Ausbeuteverluste noch erhöhen.

Aufgabe der vorliegenden Erfindung war es, Hexamethylen-diisocyanat-1,6 und/oder isomere aliphatische Diisocyanate mit 6 Kohlenstoffatomen im Alkylenrest – im folgenden insgesamt abgekürzt HDI genannt – mit hoher Selektivität in grossen Raum-Zeit-Ausbeuten kostengünstig auf einfache Weise ohne Verwendung von kostspieligen oder sicherheitsgefährdenden Ausgangs- oder Hilfsstoffen herzustellen.

Diese Aufgabe wurde gelöst durch Überführung von Hexamethylen-diamin-1,6 und/oder isomeren aliphatischen Diaminen mit 6 Kohlenstoffatomen im Alkylenrest – im folgenden insgesamt abgekürzt HDA genannt – in Hexamethylen-dialkylurethane-1,6 und/oder isomere aliphatische Dialkylurethane mit 6 Kohlenstoffatomen im Alkylenrest – im folgenden insgesamt abgekürzt mit HDU bezeichnet – und deren thermische Spaltung in HDI und Alkohole.

Erfindungsgegenstand ist somit ein Mehrstufenverfahren zur Herstellung von HDI, das dadurch gekennzeichnet ist, dass man

a) HDA mit Harnstoff und Alkohol in Gegenwart von Di-alkylcarbonaten und/oder Carbamidsäurealkylestern sowie gegebenenfalls Katalysatoren zu HDU umsetzt und das entstehende Ammoniak gleichzeitig abtrennt;

b) aus der erhaltenen Reaktionsmischung den Alkohol, die Dialkylcarbonate und/oder Carbamidsäurealkylester abtrennt und vorzugsweise in die Reaktionsstufe (a) zurückführt;

c) das HDU in einem Verdampfer bei Temperaturen von 200 bis 300 °C und einem Druck von 0,1 bis 200 mbar verdampft;

d) die Dämpfe bei Temperaturen von über 300 °C und einem Druck von 0,1 bis 200 mbar in einem Spaltreaktor in HDI und Alkohol thermisch spaltet und

e) die Spaltprodukte fraktioniert kondensiert.

Nach einer bevorzugten Ausführungsform des Verfahrens wird die erhaltene Reaktionsmischung (b) aus HDU, Dialkylcarbonat und/oder Carbamidsäurealkylester, Alkohol und gegebenenfalls Oligoharnstoff-polyurethanen zweistufig getrennt, wobei

i) in der ersten Stufe der Alkohol bis auf einen Restalkoholgehalt von 1 bis 30 Gew.-%, bezogen auf das Gesamtgewicht der Reaktionsmischung (b) abdestilliert und in die Reaktionsstufe (a) zurückgeführt wird, und

ii) in der zweiten Stufe der restliche Alkohol, das Dialkylcarbonat und/oder der Carbamidsäurealkylester durch Strippen mit Inertgas vom HDU und gegebenenfalls Oligoharnstoff-polyurethanen abgetrennt und in die Reaktionsstufe (a) zurückgeführt werden.

Nach dem erfindungsgemässen Verfahren kann HDI technisch problemlos mit sehr guten Ausbeuten hergestellt werden. Vorteilhaft bei dem Mehrstufenverfahren ist insbesondere, dass die eingesetzten und intermediär gebildeten Dialkylcarbonate und/oder Carbamidsäurealkylester und der Alkohol ohne zusätzliche kostspielige Reinigungs- und Rückgewinnungsprozesse in die Reaktionsstufe (a) zurückgeführt und wiederverwendet werden können. Die Anwesenheit der Dialkylcarbonate und/oder Carbamidsäurealkylester führt ausserdem zur Erzielung hoher HDU-Selektivitäten.

Als isomere aliphatische Diisocyanate mit 6 Kohlenstoffatomen im Alkylenrest seien insbesondere 2-Methyl-pentamethylen-1,5-diisocyanat und 2-Ethyl-tetramethylen-1,4-diisocyanat genannt. Das erfindungsgemässe Verfahren findet somit vorzugsweise Anwendung zur Herstellung der zwei obengenannten Isomeren und insbesondere von Hexamethylen-diisocyanat-1,6 sowie Gemische aus diesen Diisocyanaten.

Rein formal betrachtet kann somit das erfindungsgemässe Verfahren schematisch durch folgende Gleichung bilanziert werden:

$$H_2N(CH_2)_6{-}NH_2 + 2\ H_2NCONH_2 \rightarrow$$

$$O{=}C{=}N{-}(CH_2)_6{-}N{=}C{=}O + 4\ NH_3$$

Zur Herstellung des HDU in der Reaktionsstufe (a) wird HDA mit Harnstoff und Alkohol im Molverhältnis 1:1,8 bis 2,5:2 bis 10, vorzugsweise 1:2,0 bis 2,3:3 bis 6, in Abwesenheit oder in Gegenwart von Katalysatoren bei Reaktionstemperaturen von 160 bis 300 °C, vorzugsweise 180 bis 250 °C und insbesondere 185 bis 240 °C und unter einem Druck, der in Abhängigkeit von dem verwendeten Alkohol zwischen 0,1 bis 60 bar, vorzugsweise 1 bis 40 bar liegt, zur Reaktion gebracht. Für diese Reaktionsbedingungen ergeben sich Reaktionszeiten von 0,5 bis 50, vorzugsweise 3 bis 15 Stunden.

Als Alkohole eignen sich prinzipiell alle aliphatischen Alkohole. Vorzugsweise wird man jedoch solche auswählen, deren Siedepunkte genügend weit vom Siedepunkt des durch die thermische Spaltung erhaltenen HDI entfernt liegen, so dass einerseits eine möglichst quantitative Trennung der Spaltprodukte HDI und Alkohol möglich ist und andererseits die erhaltenen HDU, gegebenenfalls neben den Oligoharnstoff-polyurethanen, möglichst unzersetzt verdampft werden können.

Aus diesen Gründen verwendet werden daher vorzugsweise Alkohole, wie z.B. Methanol, Ethanol, n-Propanol, n-Butanol, iso-Butanol, n-Pentanol, iso-Pentanol, n-Hexanol oder Gemische der genannten Alkohole und insbesondere n- und/oder iso-Butanol.

Wie bereits dargelegt wurde, wird die Umsetzung in der Reaktionsstufe (a) in Gegenwart von Dialkylcarbonaten in einer Menge von 1 bis 30 Mol-% vorzugsweise 5 bis 25 Mol-% oder Carbamidsäurealkylestern in einer Menge von 1 bis 20 Mol-%, vorzugsweise von 5 bis 15 Mol-%, bezogen auf HDA durchgeführt. Vorzugsweise verwendet werden jedoch Mischungen aus Dialkylcarbonaten und Carbamidsäurealkylestern in den genannten Mengenverhältnissen. Als Dialkylcarbonate und/oder Carbamidsäureester setzt man bevorzugt solche ein, deren Alkylreste dem Alkylrest des verwendeten Alkohols entsprechen.

Zur Erhöhung der Reaktionsgeschwindigkeit können die HDU in Gegenwart von Katalysatoren hergestellt werden. Diese werden zweckmässigerweise in Mengen von 0,1 bis 20 Gew.-%, vorzugsweise 0,5 bis 10 Gew.-%, und insbesondere 1 bis 5 Gew.-%, bezogen auf das Gewicht des HDA, verwendet. Als Katalysatoren eignen sich anorganische oder organische Verbindungen, die ein oder mehrere Kationen, vorzugsweise ein Kation von Metallen der Gruppen IA, IB, IIA, IIB, IIIA, IIIB, IVA, IVB, VA, VB, VIB, VIIB und VIIIB des Periodensystems, definiert gemäss Handbook of Chemistry and Physics 14th. Edition, publiziert von Chemical Rubber Publishing Co., 23 Superior Ave. N.E., Cleveland, Ohio, enthalten, beispielsweise Halogenide, wie Chloride und Bromide, Sulfate, Phosphate, Nitrate, Borate, Alkoholate, Phenolate, Sulfonate, Oxide, Oxidhydrate, Hydroxide, Carboxylate, Chelate, Carbonate und Thio- oder Dithiocarbamate. Beispielhaft genannt seien die Kationen folgender Metalle: Lithium, Natrium, Kalium, Magnesium, Calcium, Aluminium, Gallium, Zinn, Blei, Bismut, Antimon, Kupfer, Silber, Gold, Zink, Quecksilber, Cer, Titan, Vanadium, Chrom, Molybdän, Mangan, Eisen, Cobalt und Nickel. Vorzugsweise Verwendung finden die Kationen von Lithium, Calcium, Aluminium, Zinn, Bismut, Antimon, Kupfer, Zink, Titan, Vanadium, Chrom, Molybdän, Mangan, Eisen und Cobalt. Die Katalysatoren können ohne erkennbare deutliche Nachteile auch in Form ihrer Hydrate oder Ammoniakate zum Einsatz kommen.

Als typische Katalysatoren seien beispielhaft folgende Verbindungen genannt: Lithiummethanolat, Lithiumethanolat, Lithiumpropanolat, Lithiumbutanolat, Natriummethanolat, Kalium-tert.-butanolat, Magnesiummethanolat, Calciummethanolat, Zinn-(II)-chlorid, Zinn-(IV)-chlorid, Bleiacetat, Bleiphosphat, Antimon-(III)-chlorid, Antimon-(V)-chlorid, Aluminium-iso-butylat, Aluminiumtrichlorid, Bismut-(III)-chlorid, Kupfer-(II)-acetat, Kupfer-(II)-sulfat, Kupfer-(II)-nitrat, Bis-(triphenylphosphinoxido)-kupfer-(II)-chlorid, Kupfermolybdat, Silberacetat, Goldacetat, Zinkoxid, Zinkchlorid, Zinkacetat, Zinkacetonylacetat, Zinkoctoat, Zinkoxalat, Zinkhexylat, Zinkbenzoat, Zinkundecylenat, Cer-(IV)-oxid, Uranylacetat, Titantetrabutanolat, Titantetrachlorid, Titantetraphenolat, Titannaphthenat, Vanadium-(III)-chlorid, Vanadiumacetonylacetat, Chrom-(III)-chlorid, Molybdän-(VI)-oxid, Molybdänacetylacetonat, Wolfram-(VI)-oxid, Mangan-(II)-chlorid, Mangan-(II)-acetat, Mangan-(III)-acetat, Eisen-(II)-acetat, Eisen-(III)-acetat, Eisenphosphat, Eisenoxalat, Eisen-(III)-chlorid, Eisen-(III)-bromid, Cobaltacetat, Cobaltchlorid, Cobaltsulfat, Cobaltnaphthenat, Nickelchlorid, Nickelacetat und Nickelnaphthenat sowie deren Gemische.

Es hat sich als vorteilhaft erwiesen, das entstehende Ammoniak sofort aus der Reaktionsmischung, beispielsweise durch Destillation abzutrennen. Die hierfür verwendete Vorrichtung, beispielsweise eine Destillationskolonne, wird bei Temperaturen von 60 bis 150 °C, vorzugsweise 65 bis 120 °C betrieben, so dass eine Belegung durch Ammoniumcarbaminat, welches in minimalen Mengen aus Ammoniak und Kohlendioxid durch Zersetzung von Harnstoff gebildet wird, vermieden werden kann.

Nach beendeter Reaktion werden aus der erhaltenen Reaktionsmischung (b) der Alkohol, die Dialkylcarbonate und/oder Carbamidsäurealkylester abgetrennt und zur Wiederverwendung bei nachfolgenden Ansätzen bereitgehalten; bei kontinuierlicher Fahrweise werden sie jedoch vorzugsweise direkt in die Reaktionsstufe (a) zurückgeführt.

Wie bereits oben dargelegt wurde, wird die Abtrennung der genannten Verbindungen vorzugsweise zweistufig durchgeführt. In der ersten Stufe wird der Alkohol bis auf einen Restalkoholgehalt von 1 bis 30 Gew.-%, vorzugsweise 2 bis 20 Gew.-%, bezogen auf das Gewicht der Reaktionsmischung (b) abdestilliert und in die Reaktionsstufe (a) zurückgeführt.

Die aufkonzentrierte Reaktionsmischung (b), die zum grössten Teil aus HDU und gegebenenfalls Oligoharnstoff-polyurethanen besteht und noch den restlichen Alkohol, Dialkylcarbonat und/oder Carbamidsäurealkylester enthält, wird in der zweiten Stufe in einer Strippkolonne mit 50 bis 5000 Liter, vorzugsweise 100 bis 1000 Liter Inertgas pro Liter aufkonzentrierter Reaktionsmischung (b) und Stunde bei Stripptemperaturen von 50 bis 200 °C, vorzugsweise 120 bis 180 °C behandelt, um den restlichen Alkohol, die Dialkylcarbonate und/oder Carbamidsäurealkylester praktisch vollständig abzutrennen. Geeignete Inertgase hierfür sind beispielsweise Stickstoff, Kohlenmonoxid, Edelgase und Erdgas. Die abgestrippten leichtersiedenden Verbindungen werden kondensiert, gegebenenfalls zwischengelagert und zur Wiederverwendung bei weiteren Ansätzen bereitgehalten. Bei kontinuierlicher Fahrweise werden sie vorzugsweise direkt in die Reaktionsstufe (a) zurückgeführt.

Der nach der Strippung erhaltene Rückstand (c), der im wesentlichen aus HDU und gegebenenfalls Oligoharnstoff-polyurethanen besteht, kann in flüssiger oder fester Form oder auch als Suspension oder Lösung in einem unter den Reaktionsbedingungen inerten Lösungsmittel in einem Verdampfer verdampft und einem nachfolgenden Spaltreaktor thermisch gespalten werden.

Nach der bevorzugten Ausführungsform des erfindungsgemässen Verfahrens wird der Rückstand (c) lösungsmittelfrei, in Form einer auf 80 bis 180 °C, vorzugsweise 100 bis 150 °C erhitzten Schmelze mit einer Dosierpumpe in den Verdampfer eingebracht.

Als Verdampfer, die bei Temperaturen von 200 bis 300 °C, vorzugsweise von 220 bis 300 °C und insbesondere von 240 bis 280 °C und unter einem Druck von 0,1 bis 200 mbar, vorzugsweise von 5 bis 100 mbar betrieben werden, haben sich insbesondere Dünnschichtverdampfer oder Wirbelschichtverdampfer bewährt. Es können jedoch auch beliebige andere Verdampfer verwendet werden, z.B. Schneckenverdampfer, A.P.-Reaktoren (Hersteller: Krauss-Maffei), Metallschlangen- oder Rührbett-Verdampfer.

Bei Verwendung von Dünnschichtverdampfern ist es zwar durch eine ausreichende Wärmezufuhr möglich, das gesamte zugeführte HDU zu verdampfen. Vorteilhafterweise wird jedoch ein Teil des zugeführten HDU gemeinsam mit gegebenenfalls vorhandenem Oligoharnstoff-polyurethanen unverdampft als Schmelze aus dem Verdampfer ausgeschleust, da man hierdurch einen bedeutenden Reinigungseffekt an der Verdampferwand erzielt. Das Gewichtsverhältnis von verdampftem zu unverdampftem HDU kann in breiten Grenzen, beispielsweise von 20:80 bis 95:5 variiert werden. Die aus dem Verdampfer ausgeschleuste Schmelze wird vorzugsweise direkt in die Reaktionsstufe (a), die Diurethanisierungsstufe, zurückgeführt.

Die HDU-Dämpfe (d) werden in den Spaltreaktor eingebracht und bei einer Temperatur von über 300 °C, vorzugsweise 310 bis 480 °C und insbesondere 360 bis 440 °C und unter vermindertem Druck, beispielsweise von 0,1 bis 200 mbar, vorzugsweise 0,1 bis 100 mbar und insbesondere 1 bis 50 mbar, diskontinuierlich oder vorzugsweise kontinuierlich in HDI und Alkohol thermisch gespalten.

Der Spaltreaktor, der im allgemeinen säulenförmig ist, kann einen Querschnitt in beliebiger Form aufweisen. Vorzugsweise verwendet man langgestreckte, zylinderförmige Spaltreaktoren. Das Verhältnis von Innendurchmesser zu Länge des Spaltreaktors beträgt im allgemeinen 1:2 bis 1:1000, vorzugsweise 1:10 bis 1:500. Die Spaltreaktoren können senkrecht oder waagrecht ausgerichtet sein und auch Zwischenlagen einnehmen. Vorzugsweise verwendet werden Röhrenöfen als Spaltreaktoren, bei denen der Rohrinnendurchmesser etwa 10 bis 100 mm und die Rohrlänge ungefähr 0,5 bis 5 m beträgt.

Zweckmässigerweise führt man die Spaltung in Gegenwart von thermisch stabilen Reaktorfüllkörpern durch. Als Füllkörper geeignet sind alle temperaturbeständigen und gasdurchlässigen Materialien, wie z.B. Perlen, Wolle, Ringe und/oder Späne aus Kohle, Stahl, Messing, Kupfer, Zink, Aluminium, Titan, Chrom, Cobalt, Nickel und/oder Quarz. Einige dieser Materialien, wie Stahl, Messing, Aluminium und Zink, haben sich besonders bewährt und werden daher bevorzugt verwendet, da sie zu besseren Spaltergebnissen führen. Hierbei ist noch ungeklärt, ob es sich um katalytische oder physikalische Effekte, wie beispielsweise eine bessere Wärmeübertragung, handelt oder ob eine synergistische Kombination beider Effekte vorliegt.

Aus dem Spaltreaktor führt man die in der Dampfphase befindlichen Dissoziationsprodukte, die nahezu ausschliessich aus HDI und Alkohol bestehen, in eine Zweistufen-Dampfkondensationsvorrichtung (e). In der ersten Kondensationsstufe, die abhängig vom Systemdruck von 0,1 bis 100 mbar bei Temperaturen von 60 bis 120 °C betrieben wird, kondensiert das HDI nahezu vollständig aus.

Bei Verwendung des bevorzugt eingesetzten Hexamethylen-dibutylurethan-1,6 wird bei einem Systemdruck von 20 bis 40 mbar zweckmässigerweise eine Kondensationstemperatur von 70 bis 100 °C eingehalten. In der zweiten Kondensationsstufe wird im wesentlichen Alkohol kondensiert, der in die Reaktionsstufe (a) zurückgeführt wird. Die Temperatur der zweiten Kondensationsstufe richtet sich nach dem Siedepunkt des zu kondensierenden Alkohols. Bei der Spaltung von Hexamethylen-dibutylurethan-1,6 wird zweckmässigerweise beim obengenannten Systemdruck eine Kondensationstemperatur von 5 bis 30 °C eingehalten. Das in der ersten Kondensationsstufe erhaltene HDI wird üblicherweise einer Reindestillation unterworfen und besitzt danach eine Reinheit von über 98 Gew.-%, vorzugsweise über 99 Gew.-%. Das hierbei anfallende Sumpfprodukt wird ebenfalls in die Reaktionsstufe (a) zurückgeführt.

Je nach Wahl der Kondensationstemperatur und in Abhängigkeit vom Systemdruck, können in der ersten Kondensationsstufe Alkohol und in der zweiten Kondensationsstufe HDI in unterschiedlichen Mengen mitkondensiert werden. Nach einer bevorzugten Ausführungsform lässt man in der zweiten Kondensationsstufe mitkondensiertes HDI mit überschüssigem Alkohol zu HDU abreagieren und führt es nach dem Abtrennen von Alkohol erneut der Verdampfung und Spaltung zu. Es ist jedoch nach einer ebenfalls bevorzugten Ausführungsform auch möglich, nach Abtrennung des Alkohols das HDU zusammen mit dem Dialkylcarbonat und/oder Carbamidsäurealkylester in die Reaktionsstufe (a) zurückzuführen.

Auf analoge Weise lässt man den in der ersten Kondensationsstufe mitkondensierten Alkohol nach der bevorzugten Ausführungsform mit überschüssigem HDI abreagieren und führt das Produktgemisch nach der destillativen Abtrennung von HDI gegebenenfalls der Verdampfung und Spaltung zu oder vorzugsweise unter Vermischung mit dem in der zweiten Kondensationsstufe erhaltenen Alkohol in die Reaktionsstufe (a) zurück.

Das nach dem erfindungsgemässen Verfahren hergestellte HDI eignet sich vorzüglich zur Herstellung von Polyurethan- oder Polyurethan-Polyharnstoff-Kunststoffen und insbesondere für lichtbeständige Polyurethanlacke und -überzüge.

Beispiel 1

In den ersten Kessel einer dreistufigen Rührkessel-Kaskade mit aufgesetzten beheizten Kolonnen und Druckhalteventil, in der sich eine Mischung aus hauptsächlich Hexamethylendibutylurethan-1,6 und n-Butanol neben Hexamethylen-oligoharnstoff-polybutylurethanen, Dibutylcarbonat und Carbamidsäurebutylester befand, gab man während einer Stunde 1,044 kg Harnstoff, 1,015 kg Hexamethylendiamin-1,6 und 0,029 kg n-Butanol und erhitzte auf 215 bis 220 °C, wobei sich ein Druck von 6 bis 8 bar einstellte. Das entstehende Ammoniak wurde über die aufgesetzten, bei 80 bis 85 °C betriebenen Kolonnen unter nahezu totalem Rückfluss des n-Butanols aus der Reaktionslösung abgetrennt. Aus dem 3. Kessel der Rührkessel-Kaskade wurde der Reaktionsaustrag in eine bei Normaldruck betriebene Füllkörperkolonne entspannt, an deren Kopfabzug ca. 3,2 kg/h n-Butanol neben Restammoniak erhalten wurden. Dieser Kopfabzug wurde direkt in den ersten Kessel der Rührkessel-Kaskade zurückgeführt. Den Destillationssumpf förderte man in eine bei 165 °C betriebene Strippkolonne, durch die ca. 250 Liter pro Stunde und Liter Reaktionsgemisch Stickstoff als Strippgas geblasen wurden. Am Kopf der Strippkolonne wurden 0,98 kg/h Gemisch aus Restbutanol, Dibutylcarbonat und Carbamidsäurebutylester erhalten. Den Sumpf der Strippkolonne förderte man ohne abzukühlen in einen auf 33 mbar evakuierten und auf 260 bis 270 °C erhitzten Dünnschichtverdampfer in der Weise, dass das Verhältnis an verdampftem Hexamethylendibutylurethan-1,6 zu ablaufender Schmelze ca. 9:1 war. Die ablaufende Schmelze vermischte man mit den Kopfprodukten der Strippkolonne und führte sie in den 2. Kessel der Rührkessel-Kaskade zurück. Die Urethandämpfe wurden in einen Spaltreaktor mit ca. 3 Liter Leervolumen, der mit V2A-Drahtnetzringen von ca. 3 mm Durchmesser gefüllt war, eindosiert. Die Temperatur im Spaltreaktor betrug durchschnittlich 410 °C. Die austretenden Spaltgase wurden in einer nachgeschalteten zweistufigen Kondensationsvorrichtung fraktioniert kondensiert. Im ersten, bei 85 °C betriebenen Kondensator fiel ein Gemisch aus 94,6 Gew.-% Hexamethylendiisocyanat-1,6 und 5,4 Gew.-% Hexamethylen-monobutylurethan-monoisocyanat an, aus dem durch eine nachgeschaltete Destillation 1,395 kg/h Hexamethylendiisocyanat-1,6 mit einer Reinheit > 99% gewonnen wurde (Selektivität bezogen auf zugeführtes Hexamethylendiamin-1,6: 95,7%). Den Sumpf der Reindestillation vermischte man mit dem im zweiten, bei 10 bis 12 °C betriebenen Kondensator erhaltenen Austrag, erhitzte diese Mischung auf 100 bis 110 °C und förderte sie in den 3. Kessel der Rührkessel-Kaskade zurück.

Beispiel 2

Es wurde analog den Angaben von Beispiel 1 verfahren, wobei jedoch in der Rührkessel-Kaskade anstelle von Hexamethylendibutylurethan-1,6 2-Methylpentamethylendibutylurethan-1,5 vorgelegt wurde. Hierzu gab man während einer Stunde 660 g 2-Methylenpentamethylendiamin-1,5, 680 g Harnstoff und 76 g n-Butanol. Am Kopf der 1. Destillationskolonne wurden in diesem Fall ca. 2,40 kg/h n-Butanol erhalten und in die Kaskade zurückgeführt. Am Kopf der Strippkolonne gewann man 1,43 kg eines Gemisches aus Dibutylcarbonat, Carbamidsäurebutylester und Restbutanol, das zusammen mit dem Verdampferablauf in den 2. Kessel der Rührkessel-Kaskade zurückgeführt wurde. Den Verdampfer belastet man so, dass das Verhältnis an verdampftem 2-Methylpentamethylendibutylurethan-1,5 zu ablaufender Schmelze etwa 46:54 betrug. Es kondensierte im ersten Kondensator ein Gemisch aus ca. 74 Gew.-% 2-Methyl-pentamethylendiisocyanat-1,5 und 26 Gew.-% Methyl-pentamethylen-monourethan-monoisocyanaten, woraus in einer nachgeschalteten Destillation 887 g/h 2-Methylpentamethylendiisocyanat-1,5 mit einer Reinheit $\geq$ 98% erhalten werden (Selektivität bezogen auf zugeführtes 2-Methyl-pentamethylendiamin-1,5: 93,6%).

**Patentansprüche**

1. Mehrstufiges Verfahren zur Herstellung von Hexamethylen-diisocyanat-1,6 und/oder isomeren aliphatischen Diisocyanaten mit 6 Kohlenstoffatomen im Alkylenrest, dadurch gekennzeichnet, dass man

a) Hexamethylen-diamin-1,6 und/oder isomere aliphatische Diamine mit 6 Kohlenstoffatomen im Alkylenrest mit Harnstoff und Alkohol in Gegenwart von Dialkylcarbonaten und/oder Carbamidsäurealkylestern sowie gegebenenfalls Katalysatoren zu Hexamethylen-dialkylurethanen-1,6 und/oder isomeren aliphatischen Dialkylurethanen mit 6 Kohlenstoffatomen im Alkylenrest umsetzt und das entstehende Ammoniak gleichzeitig abtrennt;

b) aus der erhaltenen Reaktionsmischung den Alkohol, die Dialkylcarbonate und/oder Carbamidsäurealkylester abtrennt und vorzugsweise in die Reaktionsstufe (a) zurückführt;

c) die Hexamethylen-dialkylurethane-1,6 und/oder isomeren aliphatischen Dialkylurethane mit 6 Kohlenstoffatomen im Alkylenrest in einem Verdampfer bei Temperaturen von 200 bis 300 °C und einem Druck von 0,1 bis 200 mbar verdampft;

d) die Dämpfe bei Temperaturen von über 300 °C und einem Druck von 0,1 bis 200 mbar in einem Spaltreaktor in Hexamethylen-diisocyanat-1,6 und/oder isomere aliphatische Diisocyanate mit 6 Kohlenstoffatomen im Alkylenrest und Alkohol thermisch spaltet und

e) die Spaltprodukte fraktioniert kondensiert.

2. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass die erhaltene Reaktionsmischung (b) aus Hexamethylen-dialkylurethanen-1,6 und/oder isomeren aliphatischen Dialkylurethanen mit 6 Kohlenstoffatomen im Alkylenrest, Dialkylcarbonat und/oder Carbamidsäurealkylester, Alkohol und gegebenenfalls Oligoharnstoff-polyurethanen zweistufig getrennt wird, wobei

i) in der ersten Stufe der Alkohol bis auf einen Restalkoholgehalt von 1 bis 30 Gew.-%, bezogen auf das Gesamtgewicht der Reaktionsmischung (b) abdestilliert und in die Reaktionsstufe (a) zurückgeführt wird, und

ii) in der zweiten Stufe der restliche Alkohol, das Dialkylcarbonat und/oder der Carbamidsäurealkylester durch Strippen mit Inertgas vom Hexamethylen-dialkylurethan-1,6 und/oder den isomeren aliphatischen Dialkylurethanen mit 6 Kohlenstoffatomen im Alkylenrest und gegebenenfalls Oligoharnstoff-polyurethanen abgetrennt und in die Reaktionsstufe (a) zurückgeführt werden.

3. Mehrstufiges Verfahren zur Herstellung von Hexamethylen-diisocyanat-1,6 und/oder isomeren aliphatischen Diisocyanaten mit 6 Kohlenstoffatomen im Alkylenrest nach den Ansprüchen 1 und 2, dadurch gekennzeichnet, dass der restliche Alkohol, das Dialkylcarbonat und/oder der Carbamidsäurealkylester in einer Stippkolonne bei Temperaturen von 50 bis 200 °C mit 50 bis 5000 l Inertgas pro Liter Reaktionsgemisch und Stunde abgetrennt werden.

4. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass man die Spaltprodukte in einer nachgeschalteten Zweistufenkondensationsvorrichtung fraktioniert kondensiert, wobei im ersten Teil der Kondensationsvorrichtung im wesentlichen Hexamethylen-diisocyanat-1,6 und/oder isomere aliphatische Diisocyanate mit 6 Kohlenstoffen im Alkylenrest, die einer nachfolgenden Reindestillation unterworfen werden, und im zweiten Teil der Kondensationsvorrichtung im wesentlichen Alkohol kondensiert werden, der zusammen mit dem bei der Reindestillation der aliphatischen Diisocyanate erhaltenen Sumpfprodukt in die Reaktionsstufe (a) zurückgeführt wird.

5. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass in der Reaktionsstufe (a) Hexamethylen-diamin-1,6 und/oder isomere aliphatische Diamine mit 6 Kohlenstoffatomen im Alkylenrest mit Harnstoff und Alkohol im Molverhältnis 1:1,8 bis 2,5:2 bis 10 zur Reaktion gebracht werden.

6. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass man in der Reaktionsstufe (a) als Alkohole n- und/oder iso-Butanol verwendet.

7. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch

gekennzeichnet, dass man in der Reaktionsstufe (a) den dem Alkohol entsprechenden Carbamidsäurealkylester in einer Menge von 1 bis 20 Mol-%, bezogen auf Hexamethylen-diamin-1,6 und/oder isomere aliphatische Diamine mit 6 Kohlenstoffatomen im Alkylenrest verwendet.

8. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass man in der Reaktionsstufe (a) das dem Alkohol entsprechende Dialkylcarbonat in einer Menge von 1 bis 30 Mol-%, bezogen auf Hexamethylen-diamin-1,6 und/oder isomere aliphatische Diamine mit 6 Kohlenstoffatomen im Alkylenrest verwendet.

9. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass das in der Reaktionsstufe (a) gebildete Ammoniak mit Hilfe einer Destillationsvorrichtung bei Temperaturen von 60 bis 150 °C von der Reaktionsmischung abgetrennt wird.

10. Mehrstufiges Verfahren zur Herstellung von Hexamethylen-diisocyanat-1,6 und/oder isomeren aliphatischen Diisocyanaten mit 6 Kohlenstoffatomen im Alkylenrest nach Anspruch 1, dadurch gekennzeichnet, dass man als Verdampfer einen Dünnschichtverdampfer verwendet und die Hexamethylen-dialkylurethane-1,6 und/oder isomeren aliphatischen Dialkylurethane mit 6 Kohlenstoffatomen im Alkylenrest und gegebenenfalls Oligoharnstoff-polyurethane so zuführt, dass 20 bis 95 Gew.-% Hexamethylen-dialkylurethane-1,6 und/oder isomere aliphatische Dialkylurethane-1,6 mit 6 Kohlenstoffatomen im Alkylenrest verdampfen und 5 bis 80 Gew.-%, zusammen mit gegebenenfalls vorliegendem Oligoharnstoff-polyurethanen ablaufen und in die Reaktionsstufe (a) zurückgeführt werden.

## Claims

1. A multiple-step process for the preparation of hexamethylene-diisocyanate-1,6 and/or isomeric aliphatic diisocyanates, where alkylene is of 6 carbon atoms, wherein

(a) hexamethylenediamine-1,6 and/or isomeric aliphatic diamines, where alkylene is of 6 carbon atoms, are reacted with urea and alcohol in the presence of a dialkyl carbonate and/or carbamic acid alkyl ester and, if desired, a catalyst to form hexamethylene-dialkylurethanes-1,6 and/or isomeric aliphatic dialkylurethanes, where alkylene is of 6 carbon atoms, while the ammonia which forms is simultaneously removed,

(b) the alcohol, dialkyl carbonate and/or carbamic acid alkyl ester are separated from the resulting reaction mixture and, preferably, returned to reaction step (a),

(c) the hexamethylene-dialkylurethanes-1,6 and/or isomeric aliphatic dialkylurethanes, where alkylene is of 6 carbon atoms, are evaporated in an evaporator at a temperature of from 200 to 300 °C and at a pressure of from 0.1 to 200 mbar,

(d) the vapors are thermally cleaved at a temperature in excess of 300 °C and at a pressure of from 0.1 to 200 mbar in a cleaving reactor into hexamethylene-diisocyanate-1,6 and/or isomeric aliphatic diisocyanates, where alkylene is of 6 carbon atoms, and alcohol, and

(e) the cleavage products are fractionally condensed.

2. A multiple-step process for the preparation of hexamethylene-diisocyanate-1,6 and/or isomeric aliphatic diisocyanates, where alkylene is of 6 carbon atoms, as claimed in claim 1, wherein the resulting reaction mixture (b) of hexamethylene-dialkylurethanes-1,6 and/or isomeric aliphatic dialkylurethanes, where alkylene is of 6 carbon atoms, dialkyl carbonate and/or carbamic acid alkyl ester, alcohol and, possibly, oligourea polyurethanes is separated in two steps, whereby

i) in the first step, the alcohol is distilled off until the residual alcohol content is from 1 to 30 percent by weight, based on the total weight of the reaction mixture (b), and the distilled alcohol is returned to reaction step (a), and

ii) in the second step, the residual alcohol, dialkyl carbonate and/or carbamic acid alkyl ester are separated from the hexamethylene-dialkylurethanes-1,6 and/or isomeric aliphatic dialkylurethanes, where alkylene is of 6 carbon atoms, and any oligourea polyurethanes by stripping with inert gas, and returned to reaction step (a).

3. A multiple-step process for the preparation of hexamethylene-diisocyanate-1,6 and/or isomeric aliphatic diisocyanates, where alkylene is of 6 carbon atoms, as claimed in claims 1 and 2, wherein the residual alcohol, dialkyl carbonate and/or carbamic acid alkyl ester are separated off in a stripping column at a temperature of from 50 to 200 °C with 50 to 5000 l of inert gas per liter of reaction mixture per hour.

4. A multiple-step process for the preparation of hexamethylene-diisocyanate-1,6 and/or isomeric aliphatic diisocyanates, where alkylene is of 6 carbon atoms, as claimed in claim 1, wherein the cleavage products are fractionally condensed in a downstream two-stage condenser, hexamethylene-diisocyanate-1,6 and/or isomeric aliphatic diisocyanates, where alkylene is of 6 carbon atoms, being primarily condensed in the first stage, which diisocyanates are subsequently subjected to a refining distillation, and alcohol being primarily condensed in the second stage of the condenser, which alcohol is returned to reaction step (a) together with the bottoms product obtained in the refining distillation of the aliphatic diisocyanates.

5. A multiple-step process for the preparation of hexamethylene-diisocyanate-1,6 and/or isomeric aliphatic diisocyanates, where alkylene is of 6 carbon atoms, as claimed in claim 1, wherein, in reaction step (a), 1.8 to 2.5 moles of urea and 2 to 10 moles of alcohol are reacted per mole of hexamethylene-diisocyanate-1,6 and/or isomeric

aliphatic diisocyanates, where alkylene is of 6 carbon atoms.

6. A multiple-step process for the preparation of hexamethylene-diisocyanate-1,6 and/or isomeric aliphatic diisocyanates, where alkylene is of 6 carbon atoms, as claimed in claim 1, wherein the alcohol used in reaction step (1) is n-propanol, n-butanol and/or isobutanol.

7. A multiple-step process for the preparation of hexamethylene-diisocyanate-1,6 and/or isomeric aliphatic diisocyanates, where alkylene is of 6 carbon atoms, as claimed in claim 1, wherein, in reaction step (a), the carbamic acid alkyl ester corresponding to the alcohol is used in an amount of from 1 to 20 mole percent, based on hexamethylene-diisocyanate-1,6 and/or isomeric aliphatic diisocyanates, where alkylene is of 6 carbon atoms.

8. A multiple-step process for the preparation of hexamethylene-diisocyanate-1,6 and/or isomeric aliphatic diisocyanates, where alkylene is of 6 carbon atoms, as claimed in claim 1, wherein, in reaction step (a), the dialkyl carbonate corresponding to the alcohol is used in an amount of from 1 to 30 mole percent, based on hexamethylene-diisocyanate-1,6 and/or isomeric aliphatic diisocyanates, where alkylene is of 6 carbon atoms.

9. A multiple-step process for the preparation of hexamethylene-diisocyanate-1,6 and/or isomeric aliphatic diisocyanates, where alkylene is of 6 carbon atoms, as claimed in claim 1, wherein the ammonia formed in reaction step (a) in separated from the reaction mixture with the aid of a distillation device at a temperature of from 60 to 150 °C.

10. A multiple-step process for the preparation of hexamethylene-diisocyanate-1,6 and/or isomeric aliphatic diisocyanates, where alkylene is of 6 carbon atoms, as claimed in claim 1, wherein an agitated thin-film evaporator is used as the evaporator, and the hexamethylene-diisocyanate-1,6 and/or isomeric aliphatic diisocyanates, where alkylene is of 6 carbon atoms, and any oligourea polyurethanes are added in such a manner that from 20 to 95 percent by weight of the hexamethylene-dialkylurethanes-1,6 and/or isomeric aliphatic dialkylurethanes, where alkylene is of 6 carbon atoms, evaporate and 6 to 80 percent by weight, together with any oligourea polyurethanes which may be present, run off and are returned to reaction step (a).

**Revendications**

1. Procédé à étapes multiples de préparation du diisocyanate-1,6 d'hexaméthylène et/ou de diisocyanates aliphatiques isomères dont le radical alkylène comporte 6 atomes de carbone, caractérisé en ce que

a) on convertit l'hexaméthylène-diamine-1,6 et/ou des diamines aliphatiques isomères dont le radical alkyle comporte 6 atomes de carbone, avec l'urée et un alcool, en présence de carbonates de dialkyle et/ou d'esters alkyliques de l'acide carbamique, comme éventuellement aussi de catalyseurs, en hexaméthylène-dialkyluréthanes-1,6 et/ou dialkyluréthanes aliphatiques isomères dont le radical alkylène comporte 6 atomes de carbone et on sépare simultanément l'ammoniac engendré,

b) on sépare l'alcool, les carbonates de dialkyle et/ou les esters alkyliques de l'acide carbamique du mélange réactionnel obtenu et on les renvoie, de préférence, à l'étape réactionnelle (a),

c) on évapore les hexaméthylène-dialkyluréthanes-1,6 et/ou les dialkyluréthanes aliphatiques isomères dont le radical alkylène comporte 6 atomes de carbone à des températures de 200 à 300 °C, sous une pression de 0,1 à 200 mbars, dans un évaporateur,

d) on scinde thermiquement les vapeurs à des températures supérieures à 300 °C et sous une pression de 0,1 à 200 mbars, en hexaméthylène-diisocyanate-1,6 et/ou diisocyanates aliphatiques isomères dont le radical alkylène comporte 6 atomes de carbone et en alcool, dans un réacteur de scission, et

e) on condense les produits de scission sous fractionnement.

2. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que l'on sépare le mélange réactionnel (b) obtenu, constitué d'hexaméthylène-dialkyluréthanes-1,6 et/ou de dialkyluréthanes aliphatiques isomères dont le radical alkylène comporte 6 atomes de carbone, de carbonate de dialkyle et/ou d'ester alkylique, de l'acide carbamique, d'alcool et éventuellement d'oligouréo-polyuréthanes, en deux stades, suivant lesquels

i) ou cours du premier stade, on sépare l'alcool jusqu'à une teneur en alcool résiduel de 1 à 30% en poids par rapport au poids global du mélange réactionnel (b), par distillation et on le renvoie à l'étape réactionnelle (a), et

ii) au cours du second stade, on sépare l'alcool résiduel, le carbonate de dialkyle et/ou l'ester alkylique de l'acide carbamique par lavage (stripage) avec un gaz inerte de l'hexaméthylène-dialkyluréthane-1,6 et/ou des dialkyluréthanes aliphatiques isomères dont le radical alkylène comporte 6 atomes de carbone et éventuellement des oligouréido-polyuréthanes et on les renvoie à l'étape réactionnelle (a).

3. Procédé à étapes multiples de préparation du diisocyanate-1,6 d'hexaméthylène et/ou de diisocyanates aliphatiques isomères dont le radical alkylène comporte 6 atomes de carbone suivant les revendications 1 et 2, caractérisé en ce que l'on sépare l'alcool résiduel, le carbonate de dialkyle et/ou l'ester alkylique de l'acide carbamique dans une colonne de stripage, à des températures de 50 à 200 °C avec 50 à 5000 l de gaz inerte par litre de mélange réactionnel et par heure.

4. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que l'on condense sous fractionnement les produits de scission dans un dispositif de condensation à deux stades (étages) subséquent, où, dans la première partie du dispositif de condensation, on condense essentiellement l'hexaméthylène-diisocyanate-1,6 et/ou des diisocyanates aliphatiques isomères dont le radical alkylène comporte 6 atomes de carbone, que l'on soumet à une distillation de purification subséquente et, dans une seconde partie du dispositif de condensation, on condense essentiellement l'alcool que l'on renvoie en même temps que le produit de fond obtenu au cours de la distillation de purification des diisocyanates aliphatiques, à l'étape réactionnelle (a).

5. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que, ou cours de l'étape réactionnelle (a), on fait réagir l'hexaméthylène-diamine-1,6 et/ou des diamines aliphatiques isomères dont le radical alkylène comporte 6 atomes de carbone, sur l'urée et l'alcool en une proportion molaire de 1:8 à 2,5:2 à 10.

6. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que l'on utilise le n- et/ou l'isobutanol à titre d'alcools dans l'étape réactionnelle (a).

7. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que, au cours de l'étape réactionnelle (a), on utilise l'ester alkylique d'acide carbamique correspondant à l'alcool en une proportion de 1 à 20% molaires par rapport à l'hexaméthylène-diamine-1,6 et/ou aux diamines aliphatiques isomères dont le radical alkylène comporte 6 atomes de carbone.

8. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que, au cours de l'étape réactionnelle (a), on utilise le carbonate de dialkyle correspondant à l'alcool en une proportion de 1 à 30% molaires par rapport à l'hexaméthylène-diamine-1,6 et/ou aux diamines aliphatiques isomères dont le radical alkylène comporte 6 atomes de carbone.

9. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que, au cours de l'étape réactionnelle (a), on sépare l'ammoniac formé à des températures de 60 à 150°C du mélange réactionnel à l'aide d'un dispositif de distillation.

10. Procédé à étapes multiples de préparation du diisocyanate-1,6 d'hexaméthylène et/ou de diisocyanates aliphatiques isomères dont le radical alkylène comporte 6 atomes de carbone suivant la revendication 1, caractérisé en ce que l'on utilise un évaporateur à couche mince à titre d'évaporateur et en ce que l'on introduit les hexaméthylène-dialkyluréthanes-1,6 et/ou les dialkyluréthanes aliphatiques isomères dont le radical alkylène comporte 6 atomes de carbone et éventuellement les oligouréo-polyuréthanes de façon à ce que de 20 à 95% en poids d'hexaméthylène-dialkyluréthanes-1,6 et/ou de dialkyluréthanes-1,6 aliphatiques isomères dont le radical alkylène comporte 6 atomes de carbone s'évaporent et que de 5 à 80% en poids de ces composés s'écoulent en même temps que les oligouréo-polyuréthanes éventuellement présents, pour être ensuite renvoyés à l'étape réactionnelle (a).